# EUROPEAN PATENT SPECIFICATION

(11) **EP 2 443 925 B1**
(45) Date of publication and mention of the grant of the patent: **05.11.2014**
(21) Application number: 11182352.2
(22) Date of filing: 22.09.2011
(51) Int. Cl.: A01N 25/10, A01N 25/34, A01N 43/40, A61K 8/02, A61K 8/49, A61Q 17/00, C11D 3/48, C11D 17/04, A01P 1/00, A61F 13/15, D06M 13/477

(54) **Polypropylene non-woven sheetlike support structure impregnated with a preparation comprising octenidine dihydrochloride**
Polypropylene Vliesgewebestruktur imprägniert mit einem Präparat enthaltend Octenidindihydrochlorid
Structure de support en forme de feuille du polypropylène non-tissé imprégnée d'une préparation comprenant de dichlorhydrate d'octénidine

(30) Priority: 22.10.2010 DE 102010049113
(43) Date of publication of application: 25.04.2012
(73) Proprietor: L'Air Liquide Société Anonyme pour l'Etude et l'Exploitation des Procédés Georges Claude, 75007 Paris (FR); Schülke & Mayr GmbH, 22851 Norderstedt (DE)
(72) Inventor: Dettmann, Andreas, 22157 Hamburg (DE); Behrends, Sabine, 25482 Appen (DE); Spuida, Thomas, 22419 Hamburg (DE); Wolff, Michael, 20251 Hamburg (DE); Reinstorff, Henning, 22527 Hamburg (DE)
(74) Representative: Conan, Philippe Claude

(56) References cited:
- EP-A1- 1 661 586
- WO-A1-97/16066
- WO-A1-2010/055122
- US-A- 4 100 324
- US-A1- 2005 118 240
- N O HÜBNER ET AL: "Octenidine Dihydrochloride, a Modern Antiseptic for Skin, Mucous Membranes and Wounds", SKIN PHARMACOLOGY AND PHYSIOLOGY, vol. 23, no. 5, 18 May 2010 (2010-05-18), pages 244-258, XP55018202, Basel ISSN: 1660-5527, DOI: 10.1159/000314699
- SCHÜLKE & MAYR GMBH ED - SCHÜLKE & MAYR GMBH: "octenilin Wundspüllösung Die schnelle und effektive Lösung zur Wundreinigung", INTERNET CITATION, December 2009 (2009-12), pages 1-4, XP002660661, Retrieved from the Internet: URL:http://www.schuelke.com/download/pdf/c de_lde_octenilin_Wundspuelloesung_fol.pdf [retrieved on 2011-10-06]
- MAHLTIG B ET AL: "Antimicrobial Sol-Gel Coatings", JOURNAL OF SOL-GEL SCIENCE AND TECHNOLOGY, KLUWER ACADEMIC PUBLISHERS, BO, vol. 32, no. 1-3, 1 December 2004 (2004-12-01), pages 219-222, XP019212887, ISSN: 1573-4846, DOI: 10.1007/S10971-004-5791-7
- G. MULLER ET AL: 'Biocompatibility index of antiseptic agents by parallel assessment of antimicrobial activity and cellular cytotoxicity' JOURNAL OF ANTIMICROBIAL CHEMOTHERAPY vol. 61, no. 6, 13 March 2008, pages 1281 - 1287, XP055070787 DOI: 10.1093/jac/dkn125 ISSN: 0305-7453

## Description

The invention relates to a textile sheetlike structure impregnated with an antimicrobial active ingredient preparation and with a support material based on polyolefin. The sheetlike structure is suitable in particular for use on animate surfaces, for example on human skin. The active ingredient preparation comprises a bispyridiniumalkane. Moreover, a kit comprising synthetic support material based on polyolefin and antimicrobial active ingredient preparation for producing such sheetlike structures is provided.

Disinfectant active ingredient preparations for hygienic hand disinfection according to EN 1500 and for disinfecting hand washing according to EN 1499 are known. Thus, for example, Schülke & Mayr GmbH, Norderstedt (Federal Republic of Germany) sells an aqueous composition for hand washing which comprises 0.3% by weight of the antimicrobially effective bispyridiniumalkane *octenidine dihydrochloride* (hereinbelow octenidine), and also inter alia glycerol and amine oxides as auxiliaries.

Moreover, wet wipes are described in the prior art for use on the human skin. For example, there are wet wipes for use with small children, for intimate care and for the cleaning and total bodycare of bed-ridden persons and those requiring full care. These wet wipes usually comprise substances which clean and care for the skin. For example, the wet wipes of the Menalind^{®} professional project series from Paul Hartmann AG, Heidenheim (Federal Republic of Germany) comprise an alcohol-free care liquid containing creatine and moisture-donating panthenol. For odour neutralization, a deodorizing composition is also present (cf. EP 401 140 B1). The deodorizing composition comprises a combination of two aldehydes. However, aldehydes are of concern as active ingredients for use on the skin from an allergological point of view.

Moreover, preparations of antimicrobial active ingredients applied to support materials for the disinfection of inanimate surfaces are known. However, support materials impregnated with active ingredient preparations may result in the adsorption of active ingredients, particularly if the active ingredients are quaternary ammonium compounds. This problem of adsorption of active ingredients on carrier materials also arises with the antimicrobially effective bispyridiniumalkanes.

EP 1 661 586 A1, which deals with the disinfection of hard surfaces, proposes, for the purpose of preventing active ingredient adsorption, the use of a support material made of plastic fibres, for example polyethylene terephthalate (PET). However, it has been found that certain active ingredients, which also include bispyridiniumalkanes, are adsorbed even when using PET. The problem of active ingredient adsorption on fibres of the support material is thus not solved by using PET. In particular, EP 1 661 586 Al reveals no teaching as to how a wet wipe for use on animate surfaces such as human skin can be produced using bispyridiniumalkanes as active ingredients.

WO 2004/000373 A1, which likewise deals with the disinfection of hard surfaces, accordingly proposes introducing specific additives which attach to the fibres of the support materials in order to prevent the adsorption of active ingredients. The additives proposed are specifically quaternary ammonium compounds, polydialkyldiallylammonium salts with acrylamide and/or acrylic acid and/or vinyl acetate and derivatives thereof. The obligatory use of these additives thus limits the options of formulating an active ingredient preparation with the lowest possible amount of quaternary ammonium compound, and is therefore not contemplated for applications on the skin.

Although, during the production of wet wipes it would in principle be possible, for the purpose of overcoming the disadvantage of the adsorption of active ingredient on the support material, to work with an excess of the active ingredient in the preparation, this procedure is problematic if the support materials impregnated with active ingredient preparations are then to be used on the skin. The increase in the concentration of the active ingredient is limited in this case on account of possible incompatibilities.

The object of the present invention was therefore to provide textile sheetlike materials for the cleaning and disinfection of animate surfaces (in particular human skin). The sheetlike materials should be impregnated with a preparation based on bispyridiniumalkanes as active ingredient, but the described adsorption of active ingredient on the carrier material should not result. Moreover, it should be possible to formulate active ingredient preparations in a variety of ways. Ultimately, the obligatory presence of additives which only serve to prevent adsorption but the presence of which offers no further advantages (as is the case for the additives in WO 2004/000373 A1) should not be necessary.

Surprisingly, it has now been found that these and further objects are achieved through the use of support materials based on polyolefins.

Accordingly, the invention relates to a textile sheetlike structure impregnated with an antimicrobial active ingredient preparation which comprises:
a) A support material, made of polypropylene non woven, and
b) An active ingredient preparation which comprises:
   b1) octenidine dihydrochloride in an amount of from 0.02% to 5% by weight,
   b3) water,
   b4) glycerol in an amount of from 0.5% to 10% by weight, and 1-(2-ethyl hexyl) glycerol ether in an amount from 0.01% to 5% by weight.

The invention is based inter alia on the fact that it has surprisingly been found that the adsorption of bispyridiniumalkanes is prevented with carrier materials based on polyolefins.

The support material is used as nonwoven. All nonwovens are suitable. Preference is given to nonwovens consolidated thermally or by water-jet methods. Particular preference is given to nonwovens consolidated by water-jet methods (spunlace), which are characterized by a particularly high absorbency.

Typical embodiments of the support material are gloves and wipes.

The typical total area of the glove is 100-2000 cm², preferably 300-1000 cm², in particular 500-850 cm², for example 640-770 cm², such as for example 690 cm².

If the carrier material is in the form of a glove, then the glove may be, for example, a mitten, three-finger glove or five-finger glove. In a preferred embodiment, however, the glove is produced as a simple mitten from the following amount of support material:

| | Width (cm) | Length (cm) |
|---|---|---|
| Preferably | 11-21 | 30-56 |
| More preferably | 13.5-18.5 | 36.5-49.5 |
| In particular | 14.5-17.5 | 39-47 |
| For example | about 16 | about 43 |

To produce such a simple mitten, the total area is typically placed one on top of the other and sewn or (thermally or adhesively) glued at the two sides in order to produce the mitten which, as an outside dimension, has approximately the stated width x half of the stated length.

If the support material is in the form of a wipe, then the typical area is 50-2000 cm², preferably 100-1000 cm², more preferably 300-900 cm², in particular 400-800 cm², for example about 600 cm². Typical sizes of the wipe are in the following ranges:

| | Width (cm) | Length (cm) |
|---|---|---|
| Preferably | 14-26 | 21-39 |
| More preferably | 17-23 | 25.5-34.5 |
| In particular | 18-22 | 27-33 |
| For example | about 20 | about 30 |

In the sheetlike structure according to the invention, no further support material is present besides a support material made of PP. Typical areal weights are > 15 g/m². Preferably, the areal weight when using PP as support material is in the range from 28-125 g/m², more preferably 45-105 g/m², in particular 55-95 g/m², such as, for example, about 75 g/m². A preferred embodiment is a wipe with the area stated above.

In the preferred embodiment in which the support material is PP nonwoven, preferred physical requirement on the material are:
(i) Areal weight: 75 ± 30 g/m², preferably 75 ± 10 g/m², in particular 75 ± 2 g/m², such as about 75 g/m²,
(ii) Thickness: 0.85 ± 0.50 mm, preferably 0.85 ± 0.250 mm, in particular 0.85 ± 0.10 mm, such as about 0.85 mm,
(iii) Breaking load, dry, longitudinal:
   230 ± 50 N/5 cm, preferably
   230 ± 30 N/5 cm, in particular
   230 ± 20 N/cm, such as about 230 N/5 cm (DIN EN 29073-2),
(iv) Breaking load, dry, transverse:
   66 ± 35 N/5 cm, preferably
   66 ± 25 N/5 cm, in particular
   66 ± 20 N/5 cm, such as about 66 N/5 cm (DIN EN 29073-2).

Moreover, the textile sheetlike structures according to the invention comprise b) an active ingredient preparation. This active ingredient preparation is preferably a single-phase preparation. Particular preference is given to active ingredient preparations which are in the form of a preparation or gel.

The active ingredient preparation present according to the invention obligatorily comprises bl) N,N'-(1,10-decanediyldi-1[4H]-pyridinyl-4-ylidene)bis(1-octanamine) dihydrochloride (octenidine dihydrochloride, hereinbelow octenidine).

A typical amount of component b1) is 0.02 to 5% by weight, preferably 0.03 to 3% by weight, in particular 0.04 to 0.5% by weight, for example 0.06 to 0.2% by weight, such as, for example, 0.08% by weight.

Besides the obligatorily prescribed components b1), b3) and b4) in one preferred embodiment, the active ingredient preparation used according to the invention comprises one or more of the following optional components: b2) one or more surfactants.

### b2) Surfactant

As optional constituent b2), cationic, anionic, amphoteric and/or nonionic surfactant, preferably amphoteric or nonionic surfactants, are present in the active ingredient preparations according to the invention.

As nonionic surfactant, it is possible to use all suitable nonionic surfactants, preference being given to (i) (fatty) alcohol ethoxylates, (ii) sorbitan esters, (iii) alkyl glycosides (in particular alkyl polyglucosides), (iv) amine oxides and (v) ethylene oxide/propylene oxide block copolymers. Particularly preferred nonionic surfactants are (iii) alkyl polyglucosides and (iv) amine oxides, in particular (iv) amine oxides.

The (i) alcohol polyalkoxylates include fatty alcohol alkoxylates, e.g. isodecyl ethoxylates with different fractions of ethylene oxide, isotridecyl ethoxylates, polyethylene glycol ethers of stearyl alcohol, lauryl alcohol and cetyl alcohol and oleyl alcohol. Here, the alcohols may have been alkoxylated with ethylene oxide, propylene oxide or any desired mixtures of ethylene oxide and propylene oxide. Alcohol polyalkoxylates are known inter alia under the names Lutensol^{®}, Marlipal^{®}, Marlox^{®}, Brij^{®} and Plurafac^{®}. Lauryl alcohol ethoxylates are particularly preferred as nonionic surfactant. Furthermore, the nonionic surfactants used are (ii) sorbitan esters, which are in most cases present as oleates, stearates, laurates and palmitates and which are referred to as polysorbates (e.g. Tween®).

Moreover, the nonionic surfactant may be a (iii) alkyl glycoside, such as an alkyl glucoside (i.e. an alkyl glycoside of glucose), more preferably a C₈- to C₂₀-alkylpolyglucose, in particular a C₈- to C₁₆-alkyl-polyglucose of a fatty alcohol, preference being given to a laurylpolyglucose, a decylpolyglucose or a mixture thereof. The carbon chain length for the cocoylpoly-glucose is 8 to 16 atoms, for the laurylpolyglucose is 12 to 16 carbon atoms and for the decylpolyglucose is likewise 8 to 16 carbon atoms.

A typical amount of alkyl glycoside is 0.03 to 10% by weight, preferably 0.06 to 5% by weight, in particular 0.1 to 2% by weight.

According to the invention, in principle all suitable amine oxides can be used as (iv) amine oxide. The amine oxides which are N-oxides of tertiary amines include aliphatic amine oxides, cyclic amine oxides (such as N-alkymorpholine oxide) and aromatic amine oxides (such as pyridine N-oxides). In one preferred embodiment, the amine oxide has the general formula

R¹R²R³N-O,

in which R¹ is methyl, ethyl or 2-hydroxyethyl, R² is methyl, ethyl or 2-hydroxyethyl, R¹ and R² together may be morpholine, R³ is alkyl having 8 to 18 carbon atoms or R⁴CONH(CH₂)ₙ, where R⁴ is alkyl having 8 to 18 carbon atoms and n is in the range from 1 to 10, preferably 1 to 5, more preferably 2 to 4, and in particular 3, and 2-hydroxyethyl may be condensed with 1 to 2000 ethylene oxide units, ethylene oxide/propylene oxide units or propylene oxide units.

Examples of amine oxides are cocamidopropylamine oxide, N-cocomorpholine oxide, decadimethylamine oxide, dimethylcetylamine oxide, dimethylcocamine oxide, dimethyl - hydr. tallow amine oxide, dimethyllauryl-amine oxide, dimethylmyristylamine oxide, (2-hydroxy-ethyl)cocamine oxide and oleamine oxide. See also "International Cosmetic Ingredient Dictionary and Handbook", 10th edition 2004, volume 3, pages 2268-2275 (Surfactants-Cleansing Agents).

In one preferred embodiment, the amine oxide is cocamidopropylamine oxide, i.e. R⁴CO is the acyl radical derived from the fatty acids of coconut oil, n = 3, and R¹ and R² are methyl. This product is sold as Rewominox B 204 by Goldschmidt, Federal Republic of Germany.

A typical amount of amine oxide is 0.03 to 10% by weight, preferably 0.06 to 5% by weight, in particular 0.1 to 2% by weight.

Likewise suitable as surfactant are amphoteric surfactants, for example betaines. Suitable betaines are described in EP 560 114 A2. Particular preference is given to cocomidopropylbetaine. A typical amount of betaine is 0.03 to 10% by weight, preferably 0.06 to 5% by weight, in particular 0.1 to 2% by weight.

Moreover, suitable surfactants are cationic surfactants, such as quaternary ammonium salts. In principle, all suitable quaternary ammonium compounds can be used according to the invention. The quaternary ammonium compound is preferably a dialkyldimethylammonium salt.

Quaternary ammonium salts used according to the invention are given by the formula [R¹R²R³(CH³)N]⁺[X]⁻, where R¹ to R³ may be identical or different and are selected from C₁ to C₃₀-alky, aralkyl, alkenyl and mixed groups, which can have one or more atoms selected from O, S, N and P, where R¹ to R³ are, for example, C₈- to C₁₈-alkyl, benzyl or methyl, preferably C₉- to C₁₈-alkyl, benzyl or methyl, such as C₁₆-alkyl, benzyl or methyl. X is an anion (of an inorganic or organic acid). In this connection, both anion and also cation of the quaternary ammonium salt may be polyvalent ions, giving rise to a stochiometry [A⁽ⁿ⁺¹⁾]ₘ[_{K}^{(m+)}]ₙ.

According to the invention, suitable quaternary ammonium salts are all quaternary ammonium salts of the aforementioned formula known in the prior art, as are disclosed, for example, in WO 00/63337, to which reference is hereby made. However, preference is given to using dialkyldimethylammonium salts, for example dialkyldimethylammonium chlorides, the alkyl chains of which are selected, independently of one another, from C₈- to C₁₈-alkyl, preferably C₉- to C₁₈-alkyl, such as C₁₆-alkyl. In the diaalkyldimethylammonium salts, one of the methyl groups may be an alkoxylated, for example ethoxylated, hydromethyl group.

Quaternary ammonium salts preferably used according to the invention are compounds of the formulae [R¹N(CH₃)₃]⁺[X]⁻, [R¹R²N(CH₃)₂]⁺[X]⁻ and [R¹R²R³(CH₃)N]⁺[X]⁻, where R¹ to R³, independently of one another, are selected from C₈- to C₁₈-alkyl and - (CH₂-CHR⁴O)ₙ-R⁵, where n is a number from 1 to 20, preferably 1 to 5, and R⁴ and R⁵, which may be identical or different, are H and/or C₁- to C₄-alkyl, preferably H.

Exemplary anions and classes of anions of the quaternary ammonium salts used according to the invention are hydroxide, sulphate, hydrogensulphate, methosulphate, lauryl sulphate, lauryl ether sulphate, cellulose sulphate, sulphamate, halide (fluoride, chloride, bromide, iodide), nitrite, nitrate, carbonate, hydrogencarbonate, phosphate, alkyl phosphate, metaphosphate, polyphosphate, thiocyanate (rhodanid), carboxylic acid salt such as benzoate, lactate, acetate, propionate, citrate, succinate, glutarate, adipate, toluenesulphonate (tosylate) and salicylate. Particularly preferred anions are chloride and propionate.

Particular preference is given to using the quaternary ammonium salts mecetronium etilsulphate (hexadecyl(ethyl)dimethylammonium ethylsulphate) and benzalkonium chloride.

The following constituents are present as component b4):
- Glycerol, in an amount of from 0.5 to 10% by weight, preferably 1 to 5% by weight, more preferably 1.5 to 3% by weight, such as 2 to 2.8% by weight, and -
   1-(2-ethylhexyl) glycerol ether in an amount of from 0.01 to 5% by weight, preferably 0.02 to 2% by weight, in particular 0.03 to 0.5% by weight, such as 0.04 to 0.06% by weight.

The effect described above, namely that absorption of bispyridiniumalkanes does not result with polyolefins as support material, is present at the typical pH values of active ingredient preparations which are contemplated according to the invention. Preferred pH values of the active ingredient preparations are in the range from 3 to 9, more preferably 4 to 8, such as 4.5 to 7, for example about 5.5. The desired pH can be adjusted, for example, with sodium lactate, citric acid or NaOH.

The antimicrobial active ingredient preparation is preferably a preparation which comprises
b1) octenidine in an amount of from preferably 0.02 to 2% by weight, in particular 0.04 to 1.5% by weight, such as 0.06 to 0.2% by weight, for example about 0.08% by weight,
b2) amine oxide, preferably coconut fatty acid amidopropyldimethylamine oxide, as surfactant, preferably in an amount of from 0.03 to 10% by weight, more preferably 0.06 to 5% by weight, in particular 0.1 to 2.0% by weight, such as about 0.2% by weight,
b3) water as solvent and
b4) allantoin, glycerol, 1-(2-ethylhexyl) glycerol ether and sodium lactate as auxiliaries,
where a preferred active ingredient preparation consists of components b1) to b4).

The antimicrobial active ingredient preparation is likewise preferably a preparation which comprises
b1) octenidine in an amount of from preferably 0.02 to 2% by weight, in particular 0.04 to 1.5% by weight, such as 0.06 to 0.2% by weight, for example 0.08% by weight,
b2) betaine, preferably cocamidopropylbetaine, as surfactant, preferably in an amount of from 0.03 to 10% by weight, more preferably 0.06 to 5% by weight, in particular 0.1 to 2.0% by weight, such as about 0.2% by weight,
b3) water as solvent and
b4) allantoin, glycerol, 1-(2-ethylhexyl) glycerol ether and sodium lactate as auxiliaries,
where a preferred active ingredient preparation consists of components b1) to b4).

Finally, it is preferred if the support material a) is impregnated with 1 to 12 times the weight of the active ingredient preparation b), preferably 1.5 to 8 times, such as 2 to 5 times (weight/weight).

The impregnated sheetlike structures according to the invention are used in the customary manner on animate surfaces, in particular human skin. Because the adsorption of bispyridiniumalkanes on the support material is prevented through the use of the polyolefins, the use of the sheetlike structures leads neither to diminished effectiveness of the active ingredient preparation, nor to incompatibilities (as could arise, for example, when using the additives according to WO 2004/000373 A1).

Moreover, the invention relates to the active ingredient preparation with a content of bispyridiniumalkane for use for the disinfection of animate surfaces, in particular human skin, where the active ingredient preparation as given above is present in the form of a textile sheetlike structure impregnated therewith. Moreover, the invention relates to bispyridiniumalkanes for use for the disinfection of animate surfaces, in particular human skin, where the bispyridiniumalkane is present in the form of an antimicrobial active ingredient preparation in a textile sheetlike structure impregnated therewith and having a support material which comprises polyolefin.

The advantages of the present invention arise in particular from the following examples. In the examples and in the description above, all of the percentages refer to the total weight of the active ingredient preparation.

### Example 1

Test design: 9 PET gloves (80 g/m²) and 12 PP wipes (75 g/m²) were impregnated with 3.25 times the weight of impregnation preparation, stored for 24 hours in stacking boxes and then squeezed using a stainless steel potato press. During the squeezing of the wipes/gloves, in each case a squeezing-out rate of ca. 30-50% of the impregnation amount originally used was advised.

| | **A** | **B** |
|---|---|---|
| Purified water | 96.875 | 96.875 |
| Octenidine | 0.050 | 0.050 |
| Glycerol 85% | 2.850 | 2.850 |
| Oxadermol | 0.125 | 0.125 |
| Allantoin | 0.10 | 0.01 |
| Citric acid | x | |
| NaOH 10% | | x |
| pH | 4.0 | 8.0 |
| | Octenidine [%] | Octenidine [%] |
| Value | 0.052 | 0.052 |
| PET gloves (comparison) | 0.040 | 0.043 |
| PP wipes | 0.048 | 0.046 |

### Example 2

Test design: In each case 10 PET gloves (80 g/m²), PP wipes (75 g/m²) and PET wipes (45 g/m²) were impregnated with 3.25 times the weight of impregnation preparation, stored for 24 hours in stacking boxes and then squeezed using a stainless steel potato press. During the squeezing of the wipes/gloves, in each case a squeezing-out rate of 30-40% of the impregnation amount originally used was advised. The experiment was repeated.

| | **C** | **D** |
|---|---|---|
| | w (%) | w (%) |
| Purified water | 96.535 | 96.535 |
| Octenidine dihydrochloride | 0.050 | 0.050 |
| Glycerol 85% | 2.850 | 2.850 |
| Oxadermol | 0.125 | 0.125 |
| Allantoin | 0.100 | 0.10 |
| Sodium lactate solution 50% | 0.340 | 0.34 |
| Total: | 100,000 | 100,000 |
| pH: | 5.5 | 5.6 |
| | Octenidine, % | Octenidine, % |
| Value | 0.051 | 0.054 |
| PET gloves (comparison) | 0.040 | 0.040 |
| PET wipes (comparison) | 0.044 | 0.045 |
| PP wipes | 0.046 | 0.048 |

## Claims

1. Textile sheetlike structure impregnated with an antimicrobial active ingredient preparation which comprises:
a) A support material, made of polypropylene non woven, and
b) An active ingredient preparation which comprises:
b1) octenidine dihydrochloride in an amount of from 0.02% to 5% by weight,
b3) water,
b4) glycerol in an amount of from 0.5% to 10% by weight, and 1-(2-ethyl hexyl) glycerol ether in an amount from 0.01% to 5% by weight.

2. Sheetlike structure according to Claim 1, **characterized in that** the active ingredient preparation also comprises:
b2) amine oxide, in an amount of from 0.03 to 10% by weight.

3. Sheetlike structure according to Claim 1, **characterized in that** the active ingredient preparation also comprises:
b2) a betaine in an amount of from 0.03 to 10% by weight.

4. Sheetlike structure according to one of the preceding claims, **characterized in that** the support material has an areal weight of 45 to 105 g/m².

## Patentansprüche

1. Textile, mit einem einen antimikrobiellen Wirkstoff enthaltenden Präparat imprägnierte, tuchähnliche Struktur, welche Folgendes umfasst:
a) ein Trägermaterial aus Polypropylen-Vlies, und
b) ein Präparat als Wirkstoff, umfassend:
b1) Octenidindihydrochlorid in einer Konzentration von 0,02 bis 5 Gew.-%.,
b3) Wasser,
b4) Glycerin in einer Konzentration von 0,5 bis 10 Gew.-% und 1-(2-Ethylhexyl)glycerinether in einer Konzentration von 0,01 bis 5 Gew.-%.

2. Tuchähnliche Struktur nach Anspruch 1,
**dadurch gekennzeichnet, dass** das Präparat als Wirkstoff ferner umfasst:
b2) Aminoxid in einer Konzentration von 0,03 bis 10 Gew.-%.

3. Tuchähnliche Struktur nach Anspruch 1,
**dadurch gekennzeichnet, dass** das Präparat als Wirkstoff ferner umfasst:
b2) ein Betain in einer Konzentration von 0,03 bis 10 Gew.-%.

4. Tuchähnliche Struktur nach einem der vorhergehenden Ansprüche, **dadurch gekennzeichnet, dass** das Trägermaterial ein Flächengewicht von 45 bis 105 g/m² hat.

## Revendications

1. Structure textile de type feuille imprégnée par une préparation d'ingrédient actif antimicrobien qui comprend :
a) un matériau de support, constitué de poly(propylène) non tissé, et
b) une préparation d'ingrédient actif qui comprend :
b1) du dichlorhydrate d'octénidine dans une quantité de 0,02 % à 5 % en poids,
b3) de l'eau,
b4) du glycérol dans une quantité de 0,5 % à 10 % en poids, et du 1-(2-éthylhexyl)glycérol éther dans une quantité de 0,01 % à 5 % en poids.

2. Structure textile de type feuille selon la revendication 1, **caractérisée en ce que** la préparation d'ingrédient actif comprend également :
b2) un oxyde d'amine, dans une quantité de 0,03 à 10 % en poids.

3. Structure textile de type feuille selon la revendication 1, **caractérisée en ce que** la préparation d'ingrédient actif comprend également :
b2) une bétaïne dans une quantité de 0,03 à 10 % en poids.

4. Structure textile de type feuille selon l'une des revendications précédentes, **caractérisée en ce que** le matériau de support a un poids surfacique de 45 à 105 g_{/}m².
